# EUROPEAN PATENT APPLICATION

(11) **EP 1 035 104 A1**
(43) Date of publication of application: **13.09.2000**
(21) Application number: 00103303.4
(22) Date of filing: 17.02.2000
(51) Int. Cl.: C07C 67/60, C07C 69/54

(54) **Process of producing methyl methacrylate**

(30) Priority: 12.03.1999 JP 6644099
(71) Applicant: MITSUBISHI GAS CHEMICAL COMPANY, INC., Tokyo 100-0005 (JP)
(72) Inventor: Nakano, Rieko, Mitsubishi Gas Chem. Company Inc., Tayuhama, Niigata-shi, Niigata-ken (JP); Takemoto, Masaki, Mitsubishi Gas Chem.Company Inc., Tayuhama, Niigata-shi, Niigata-ken (JP); Nakamura, Goh, Mitsubishi Gas Chem. Company Inc., Tayuhama, Niigata-shi, Niigata-ken (JP)
(74) Representative: Gille Hrabal Struck Neidlein Prop Roos

(57) **Abstract**

Biacetyl contained in methyl methacrylate produced by known methods is efficiently converted to a substance on contacting an aliphatic compound having at least three, in total, amino and/or imino groups. Since the substance is easily separable from methyl methacrylate by usual distillation technique, methyl methacrylate substantially free from biacetyl is obtained. The aliphatic compound is preferably a polyethylenepolyamine such as diethylenetriamine and triethylenetetramine.

## Description

### FIELD OF THE INVENTION

The present invention relates to a low cost process of efficiently producing methyl methacrylate substantially free from a coloring substance such as biacetyl (CH₃COCOCH₃).

### BACKGROUND OF THE INVENTION

Methyl methacrylate has been widely used in manufacturing optical materials such as organic glass, optical fibers, various coating materials, materials for contact lenses. Methyl methacrylate has been mass-produced in industrial scale formerly by ACH (acetone cyanohydrin) method accompanying by-production of ammonium sulfate, and subsequently by various methods such as C₄ oxidation method, modified ACH method, method of oxidative esterification of methacrolein, etc.

Methyl methacrylate produced by the above methods inevitably contains a non-negligible amount, usually more than one hundred ppm, of coloring substance which is by-produced during the production of methyl methacrylate due to exposure to oxidative atmosphere and high temperatures. Such a coloring substance extremely reduces the commercial value of methyl methacrylate as the optical materials. In particular, the contamination with coloring substance should be avoided in optical fibers because the coloring substance absorbs lights having certain wave-length and inhibits transmission thereof. Therefore, the following proposals have been made to remove the coloring substance from methyl methacrylate:
(1) method of distilling off the coloring substance at a high reflux ratio using a tray tower,
(2) method of adsorbing the coloring substance on activated carbon,
(3) method of adsorbing the coloring substance by passing methyl methacrylate through a solid adsorbent such as alumina as disclosed in Japanese Patent Publication No. 60-18964,
(4) method of treating methyl methacrylate with an aromatic diamine as proposed in EP 206,230, and
(5) method of treating methyl methacrylate with a non-aromatic 1,2-diamine as proposed in Japanese Patent Application Laid-Open No. 8-169862.

However, the complete removal of the coloring substance by distillation is immensely energy-consuming because the coloring substance such as biacetyl has a low relative volatility to methyl methacrylate. The removal by treating methyl methacrylate with activated carbon or alumina is less effective and no satisfactory removal is obtained. The method of using an aromatic diamine is unfavorable for economical and safety reasons because a large excess of expensive and injurious aromatic diamine with respect to the coloring substance should be used. The method of using a non-aromatic diamine is costly because an amount of non-aromatic diamine as large as 10 times by mole or more with respect to biacetyl is required, as taught by Japanese Patent Application Laid-Open No. 8-169862. In addition, the complete separation of the non-aromatic diamine from methyl methacrylate requires a large number of distillation trays and a large quantity of energy because ethylenediamine, a typical non-aromatic diamine, has the boiling point of 117°C which is very close to that of methyl methacrylate.

Thus, an object of the present invention is to provide a process of efficiently producing methyl methacrylate free from the coloring biacetyl.

### SUMMARY OF THE INVENTION

The present invention provides a process of producing methyl methacrylate substantially free from biacetyl, in which at least one of a step for synthesizing methyl methacrylate, a step of extracting methyl methacrylate and a step of distilling methyl methacrylate employed in known methods of producing methyl methacrylate is carried out in the presence of an aliphatic compound having at least three, in total, amino groups (-NH₂) and/or imino groups (-NH-) in its molecule.

### DETAILED DESCRIPTION OF THE INVENTION

The present invention will be described below in more detail.

In the present invention, biacetyl contained in methyl methacrylate as a coloring substance in a small amount is reacted with an aliphatic compound having at least three, in total; primary and/or secondary amino groups in its molecule, thereby converting biacetyl to a substance having chemical properties quite different from those of methyl methacrylate without substantially losing the amount of methyl methacrylate. Namely, biacetyl is converted into a substance easily separable from methyl methacrylate by usual distillation, thereby enabling a sufficient removal of biacetyl and easy production of methyl methacrylate substantially free from the coloring biacetyl. Generally, biacetyl is converted into a heterocyclic compound upon contacting the aliphatic compound.

The aliphatic compound having at least three, in total, primary and/or secondary amino groups in its molecule is preferably a polyethylenepolyamine represented by the following formula (I): 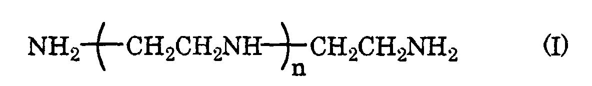 wherein suffix "n" is an integer from 1 to 4.

Of the aliphatic compounds represented by the formula (I), preferred are diethylenetriamine (boiling point: 206.7°C) and triethylenetetramine (boiling point: 277.5°C), and diethylenetriamine is most preferably used. To prevent the aliphatic compound from remaining in the final methyl methacrylate (boiling point: 101°C), the boiling point of the aliphatic compound is required to be sufficiently apart from that of methyl methacrylate so that the aliphatic compound may be easily separated from methyl methacrylate after completing the conversion of biacetyl to easily separable substance. Specifically, the boiling point of the aliphatic compound is preferably 150°C or higher.

The aliphatic compound may be added at any stages of known methods of producing methyl methacrylate, for example, at least one of stages such as a step for synthesizing methyl methacrylate, a step for extracting methyl methacrylate and a step for distilling methyl methacrylate. The amount of the aliphatic compound to be added is 1 to 50 mol, preferably 1 to 10 mol per one mol of biacetyl to be removed.

After adding the aliphatic compound to methyl methacrylate, the aliphatic compound is contacted with biacetyl preferably under stirring the resultant mixture. A contacting time for 5 to 60 minutes is usually sufficient for reducing the biacetyl content in methyl methacrylate to 0.5 ppm or less if the treating temperature is 40°C or higher. However, since the contacting time varies depending on a reactor, a storage container and a residence time in a distillation apparatus, a contacting time longer than the above range may be employed without causing any disadvantages.

After completing the conversion of biacetyl to easily separable substance, methyl methacrylate is isolated from the mixture by known methods such as fractional distillation, etc., thereby obtaining methyl methacrylate substantially free from biacetyl, i.e., methyl methacrylate containing 0.5 ppm or less of biacetyl.

The present invention may be applied to methyl methacrylate produced by any known methods, and also applied to removal of biacetyl from the commercially distributed methyl methacrylate.

Since methyl methacrylate obtained by the present invention is practically colorless, resins and resin compositions made thereof are excellent in optical properties and extremely suitable as optical materials.

As described above, the present invention provides practically colorless methyl methacrylate by easy process, and the process of the present invention is of great industrial value.

The present invention will be described in further detail by way of the following Examples. However, it should be noted that the following Examples are not intended to limit the invention thereto.

### Example 1

Into a flask equipped with a reflux condenser and a stirring device, were charged 50 g of methyl methacrylate containing 150 ppm biacetyl. The charged methyl methacrylate had yellow color, and its APHA value determined by visually comparing with standard colored liquids was 100 or more. After adding to the flask diethylenetriamine in an amount 4.8 times by mole with respect to biacetyl concentration in methyl methacrylate, the resultant test mixture was heated to 50°C and stirred to follow the change with time of the biacetyl concentration in methyl methacrylate. The amount of biacetyl was determined by gas chromatographic analysis with 0.5 ppm lower detection limit. The results of analysis showed that the concentration of biacetyl was reduced to a level lower than the lower detection limit after 5 minutes of adding diethylenetriamine. Separately, the test mixture was distilled under 200 mmHg using a batch-wise vacuum distillation apparatus having Vigreux fractionating column of 20 cm high. The concentration of biacetyl in the methyl methacrylate distillate was also lower than the lower detection limit and the APHA value was 5 or less. The total nitrogen content in the distillate measured by emission spectroscopy was lower than the lower detection limit (< 1 ppm).

### Example 2

The procedures of Example 1 were repeated except for changing the addition amount of diethylenetriamine to 1.05 times by mole. The results showed that the biacetyl concentration in methyl methacrylate was 3.4 ppm after five minutes, 0.9 ppm after 30 minutes and lower than the lower detection limit after 60 minutes.

### Example 3

The procedures of Example 1 were repeated except for adding 1.5 times by mole of triethylenetetramine in place of diethylenetriamine. The results showed that the biacetyl concentration in methyl methacrylate was 1.1 ppm after five minutes and lower than the lower detection limit after 30 minutes.

### Comparative Example 1

The procedures of Example 1 were repeated except for adding 2.2 times by mole of ethylenediamine in place of diethylenetriamine. The results showed that the biacetyl concentration in methyl methacrylate was 4.7 ppm after five minutes, 3.0 ppm after 30 minutes and 1.2 ppm after 60 minutes. Thus, the addition of ethylenediamine failed to completely remove biacetyl. Separately, the test mixture was distilled in the same manner as in Example 1. The results of gas chromatographic analysis and emission spectroscopic analysis of the methyl methacrylate distillate showed that the biacetyl concentration was 1.3 ppm and the total nitrogen content was 30 ppm.

## Claims

1. A process of producing methyl methacrylate substantially free from biacetyl, which comprises:
reacting biacetyl contained in methyl methacrylate with an aliphatic compound having at least three, in total, amino and/or imino groups in its molecule, thereby converting biacetyl to a substance easily separable from methyl methacrylate; and
removing the substance.

2. The process according to claim 1, wherein the boiling point of said aliphatic compound is 150°C or higher.

3. The process according to claim 1 or 2, wherein said aliphatic compound is a polyethylenepolyamine represented by the following formula: 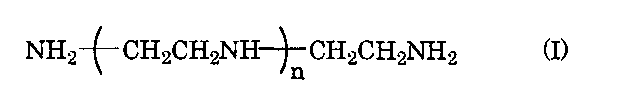 wherein suffix "n" is an integer from 1 to 4.

4. The process according to any one of claims 1 to 3, wherein said aliphatic compound is diethylenetriamine or triethylenetetramine.

5. The process according to any one of claims 1 to 4, wherein said aliphatic compound is added in an amount of 1 to 50 mol per one mol of biacetyl to be removed.

6. The process according to any one of claims 1 to 5, wherein said aliphatic compound is brought into contact with biacetyl at a temperature of 40°C or higher for at least 5 minutes.

7. The process according to any one of claims 1 to 6, wherein the biacetyl concentration in said methyl methacrylate substantially free from biacetyl is 0.5 ppm or less.

8. A resin obtained by polymerizing said methyl methacrylate substantially free from biacetyl produced by a method according to any one of claims 1 to 7.

9. A resin composition comprising the resin according to claim 8.
